# EUROPEAN PATENT SPECIFICATION

(11) **EP 2 732 772 B1**
(45) Date of publication and mention of the grant of the patent: **12.06.2019**
(21) Application number: 12192668.7
(22) Date of filing: 14.11.2012
(51) Int. Cl.: A61B 17/072

(54) **Multi-use loading unit**
Mehrzweckladeeinheit
Unité de chargement à usages multiples

(43) Date of publication of application: 21.05.2014
(73) Proprietor: Covidien LP, Mansfield, MA 02048 (US)
(72) Inventor: Kostrzewski, Stanislaw, Newtown, CT 06470 (US); Aranyi, Ernest, Easton, CT 06612 (US); Scirica, Paul A, Huntington, CT 06484 (US); Powers, William, Cheshire, CT 06410 (US)
(74) Representative: Maschio, Antonio

(56) References cited:
- EP-A1- 2 165 654
- EP-A1- 2 253 280
- EP-A2- 0 754 433
- EP-A2- 2 586 382
- WO-A1-2008/039238
- CA-A1- 2 773 414
- US-A- 4 892 244
- US-A- 5 752 644
- US-A1- 2012 138 660

## Description

### BACKGROUND

### Technical field

The present disclosure relates generally to instruments for surgically joining tissue and, more specifically, to a multi-use loading unit for use with surgical instruments.

### Background of Related Art

Various types of surgical instruments used to surgically join tissue are known in the art, and are commonly used, for example, for closure of tissue or organs in transection, resection, anastomoses, for occlusion of organs in thoracic and abdominal procedures, and for electro surgically fusing or sealing tissue.

One example of such a surgical instrument is a surgical stapling instrument, which may include an anvil assembly, a cartridge assembly for supporting an array of surgical staples, an approximation mechanism for approximating the cartridge and anvil assemblies, and a firing mechanism for ejecting the surgical staples from the cartridge assembly.

Using a surgical stapling instrument, it is common for a surgeon to approximate the anvil and cartridge members. Next, the surgeon can fire the instrument to emplace staples in tissue. Additionally, the surgeon may use the same instrument or a separate instrument to cut the tissue adjacent or between the row(s) of staples. US patent application publication number US 2012/138660 A1 and European patent application publication number EP 2165654 A1 both disclose a surgical stapling device comprising a cartridge body mounted in a cartridge tray which is in turn received in a channel. The channel comprises an anvil cam slot that pivotably receives an anvil pivot.

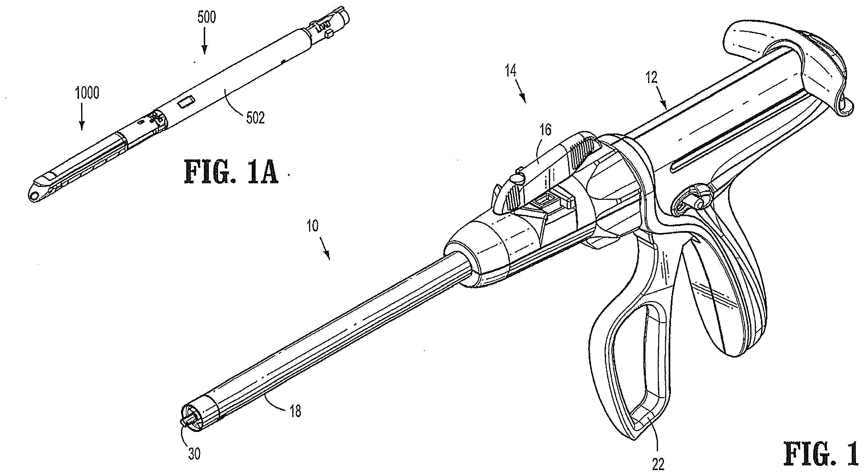

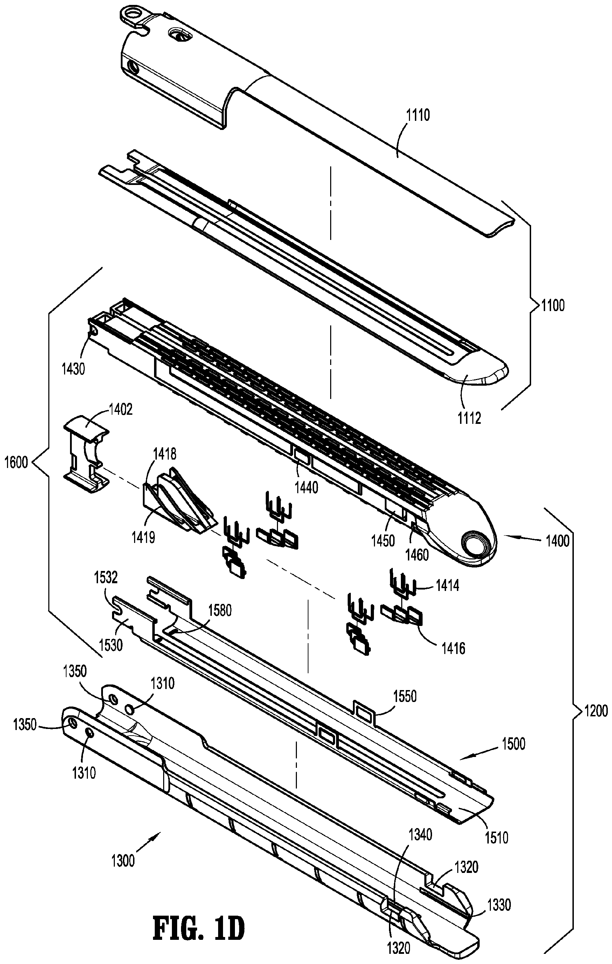

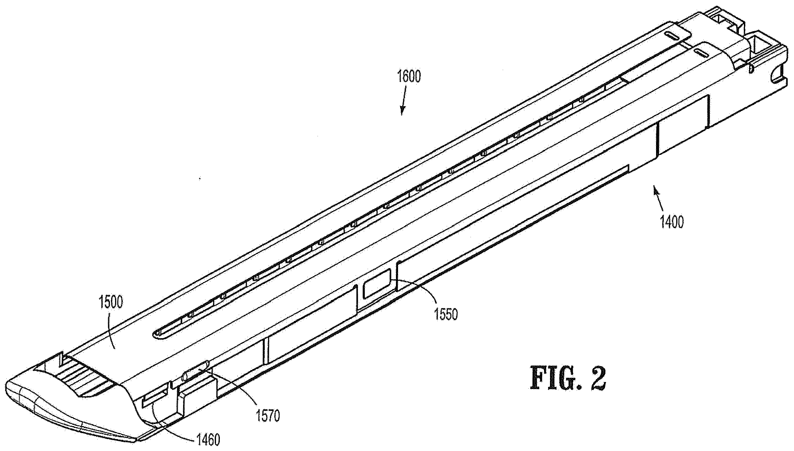

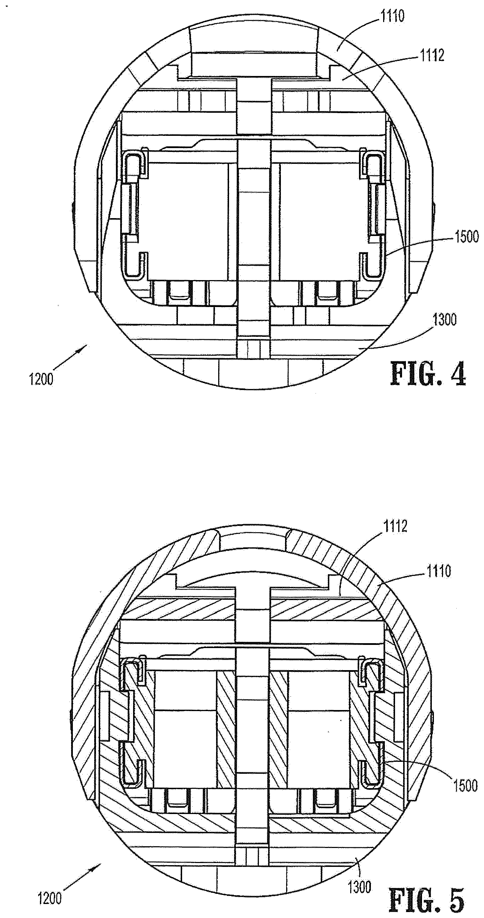

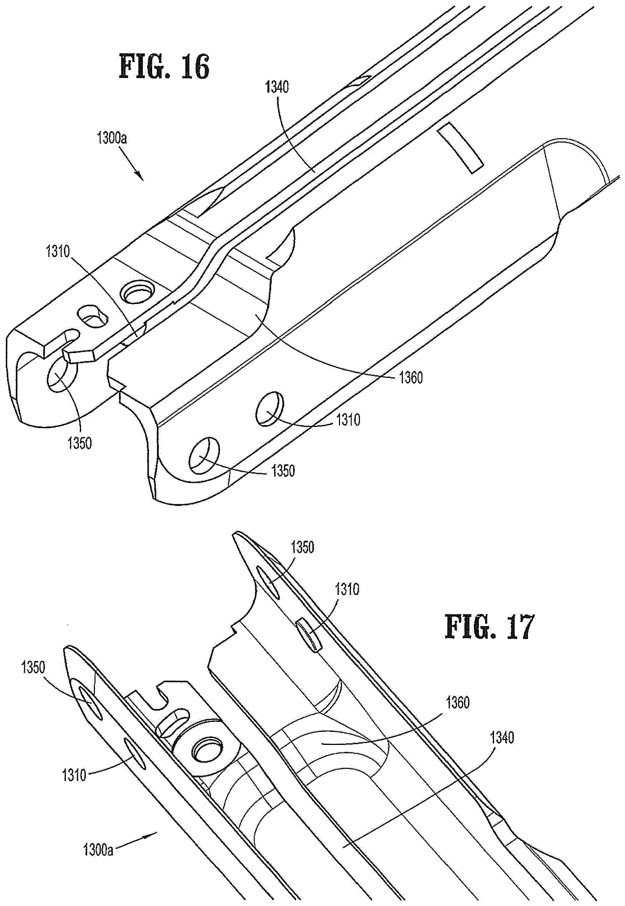

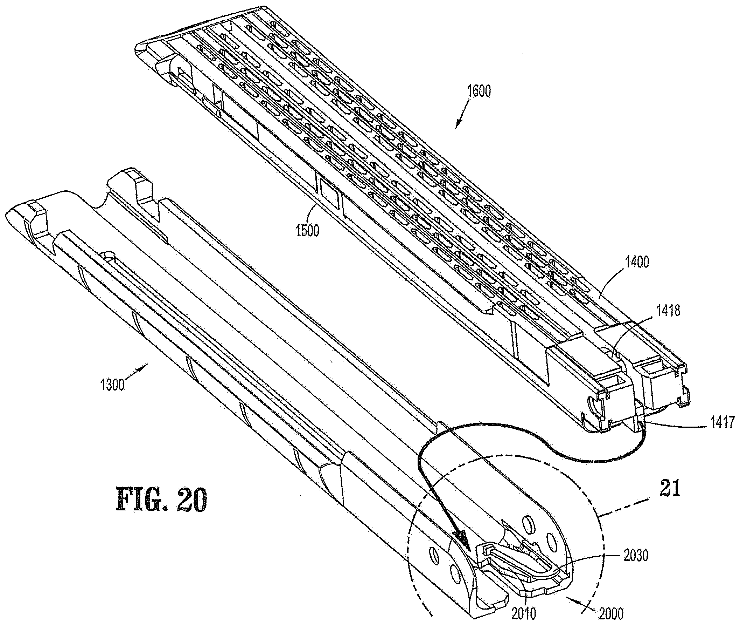

The invention is disclosed in figs. 1D-6, 10, 12, 16, 17, 20 and the corresponding passages in the description. The present disclosure relates to a surgical instrument having a channel and a removable assembly disposed in releasable engagement with the channel. The removable assembly includes a cartridge body and a support plate. The cartridge body is configured to house a plurality of fasteners or staples therein and includes an engagement structure disposed adjacent a proximal end thereof. The support plate is configured to mechanically engage the cartridge body and includes an engagement structure disposed adjacent a proximal end thereof. The engagement structure of the cartridge body is configured for longitudinal alignment with the engagement structure of the support plate. The engagement structure of the cartridge body and the engagement structure of the support plate are configured to mechanically engage engagement structure of the channel when the removable assembly is engaged with the channel.

According to the invention the engagement structure of the channel includes at least one raised boss. In disclosed embodiments, the engagement structure of the cartridge body includes a U-shaped recess, and/or the engagement structure of the support plate includes a U-shaped recess. In disclosed embodiments, the U-shaped recesses of the cartridge body and the support plate include a proximally-facing opening.

In disclosed embodiments, the channel includes a longitudinally-extending slot disposed adjacent a distal end thereof, and the support plate includes an outwardly-extending finger configured to releasably engage the longitudinally-extending slot of the channel.

In disclosed embodiments, the support plate includes an inwardly-extending finger disposed on a distal portion thereof. Here, the inwardly-extending finger is configured to releasably engage a groove disposed on a distal portion of the cartridge body.

In disclosed embodiments, the support plate includes a proximal protrusion disposed adjacent a proximal end thereof. The proximal protrusion is configured to help prevent an actuation sled from prematurely translating distally with respect to the cartridge body.

In certain embodiments, the channel is part of a removable loading unit that includes an anvil assembly.

In a further aspect of the present disclosure, a loading unit for a surgical instrument has an anvil assembly, a channel, and a cartridge assembly. The channel has a boss disposed adjacent a proximal end thereof. The cartridge assembly and anvil assembly are pivotable with respect to one another. The cartridge assembly includes a support plate, and a cartridge body. The support plate is configured to releasably engage the channel and includes a recess disposed adjacent a proximal end thereof. The cartridge body is configured to releasably engage the support plate and is configured to house a plurality of fasteners or staples therein. The cartridge body includes a recess disposed adjacent a proximal end thereof. The recess of the cartridge body is configured for longitudinal alignment with the recess of the support plate. At least one of the recesses of the cartridge body and the support plate is configured to mechanically engage the boss of the channel when the support plate is engaged with the channel.

In disclosed embodiments, the recess of the cartridge body includes a U-shaped recess and/or the recess of the support plate includes a U-shaped recess. In such embodiments, the U-shaped recesses of the cartridge body and the support plate include a proximally-facing opening.

In disclosed embodiments, the channel includes a longitudinally-extending slot disposed adjacent a distal end thereof, and the support plate includes an outwardly-extending finger configured to releasably engage the longitudinally-extending slot of the channel.

In disclosed embodiments, the support plate includes an inwardly-extending finger disposed on a distal portion thereof. The inwardly-extending finger is configured to releasably engage a groove disposed on a distal portion of the cartridge body.

In disclosed embodiments, the support plate includes a proximal protrusion disposed adjacent a proximal end thereof. The proximal protrusion is configured to help prevent an actuation sled from prematurely translating distally with respect to the cartridge body.

In certain embodiments, the loading unit includes a body portion to which the cartridge assembly and anvil assembly are attached the body portion being attachable to the elongate member of a surgical instrument.

The present disclosure also relates to a surgical instrument having a channel and comprising a cartridge assembly, a drive member and a lockout mechanism. The drive member is configured to travel in a distal direction. The lockout mechanism is configured to prevent longitudinal translation of the drive member. The lockout mechanism comprises a latch and a spring. The latch is disposed in mechanical cooperation with the channel and is laterally movable from an initial position to a blocking position. The spring is configured to bias the latch into the blocking position in which a shaped surface of the latch obstructs the distal movement of the drive member when the latch is in the blocking position.

. In disclosed embodiments, the latch is pivotable with respect to the cartridge assembly.

In disclosed embodiments, the latch includes a hook configured to engage a portion of the drive member to prevent distal translation of the drive member.

In disclosed embodiments, the latch includes a camming surface, and wherein when the drive member translates proximally into contact with the camming surface, the latch pivots away from its blocking position.

In disclosed embodiments, the surgical instrument comprises a sled configured for longitudinal translation with respect to at least a portion of the cartridge assembly. The sled includes a tail portion that is configured to abut a portion of the latch when the sled is adjacent its proximal-most position. The tail portion of the sled is configured to prevent the latch from moving into its blocking position.

The cartridge assembly may include-a cartridge body defining a longitudinal slot. The drive member travels along the longitudinal slot in the distal direction. The shaped surface of the latch is substantially aligned with the longitudinal slot when the latch is in the blocking position.

### BRIEF DESCRIPTION OF FIGURES

Various embodiments of the presently disclosed surgical instrument are disclosed herein with reference to the drawings, wherein:
Figure 1 is a perspective view of a surgical stapling instrument without a loading unit connected thereto in accordance with the present disclosure;
Figure 1A is a perspective view of a loading unit in accordance with the present disclosure;
Figure 1B is a perspective view of a tool assembly of the loading unit of Figure 1A;
Figure 1C is a perspective view of a cartridge assembly of the loading unit of Figure 1A;
Figure 1D is an assembly view of the tool assembly of Figure 1B;
Figure 2 is a bottom perspective view of a portion of the tool assembly of Figure 1B;
Figure 3 is a perspective view of a portion of the tool assembly of Figure 1B;
Figures 4 and 5 are transverse cross-sectional views of portions of the tool assembly of Figure 1B;
Figure 6 is a perspective view of a proximal portion of a channel of the tool assembly of Figure 1B;
Figure 7 is a perspective view of a distal portion of the channel of the tool assembly of Figure 1B;
Figure 8 is a transverse cross-sectional view of portion of the tool assembly of Figure 1B;
Figure 9 is a perspective view of the tool assembly of Figure 1B;
Figure 10 is a perspective view of a support plate of the tool assembly of Figure 1B;
Figure 11 is a perspective view of a distal portion of a cartridge body of the tool assembly of Figure 1B;
Figure 12 is a perspective view of a proximal portion of the cartridge body of the tool assembly of Figure 1B;
Figure 13 is a perspective view of a portion of a tool assembly of the present disclosure including another embodiment of a channel;
Figures 14 and 15 are perspective views of the channel of Figure 13;
Figures 16 and 17 are perspective views of different portions of the channel of Figure 13;
Figure 18 is a perspective view of a tool assembly of the present disclosure including a lockout mechanism;
Figure 19 is an enlarged perspective view of the lockout mechanism of the present disclosure engaged with a portion of the tool assembly;
Figure 20 is a perspective assembly view of portions of the tool assembly includes the lockout assembly;
Figure 21 is a perspective view of the lockout mechanism engaged with the channel;
Figure 22 is a perspective assembly view of the lockout mechanism and a portion of the channel;
Figure 23 is an assembly view of the removable assembly of an embodiment of the present disclosure;
Figure 24 is a perspective view of a latch of the lockout mechanism of the present disclosure;
Figure 25 is a perspective view of a sled of the present disclosure;
Figure 26 is a top view of the cartridge assembly taken along line 26-26 of Figure 18 and illustrating the lockout mechanism, and the drive member and sled in their original positions;
Figure 27 is an enlarged view of the area indicated in Figure 26;
Figures 28-31 are top views of a portion of the cartridge assembly showing the drive member, sled, and latch in various positions;
Figures 32-35 are perspective views of a second embodiment of a lockout mechanism in accordance with an embodiment of the present disclosure.

### DETAILED DESCRIPTION

Embodiments of the presently disclosed surgical instrument, loading unit and tool assembly for use therewith, are described in detail with reference to the drawings, wherein like reference numerals designate corresponding elements in each of the several views. As is common in the art, the term 'proximal" refers to that part or component closer to the user or operator, e.g., surgeon or physician, while the term "distal" refers to that part or component farther away from the user.

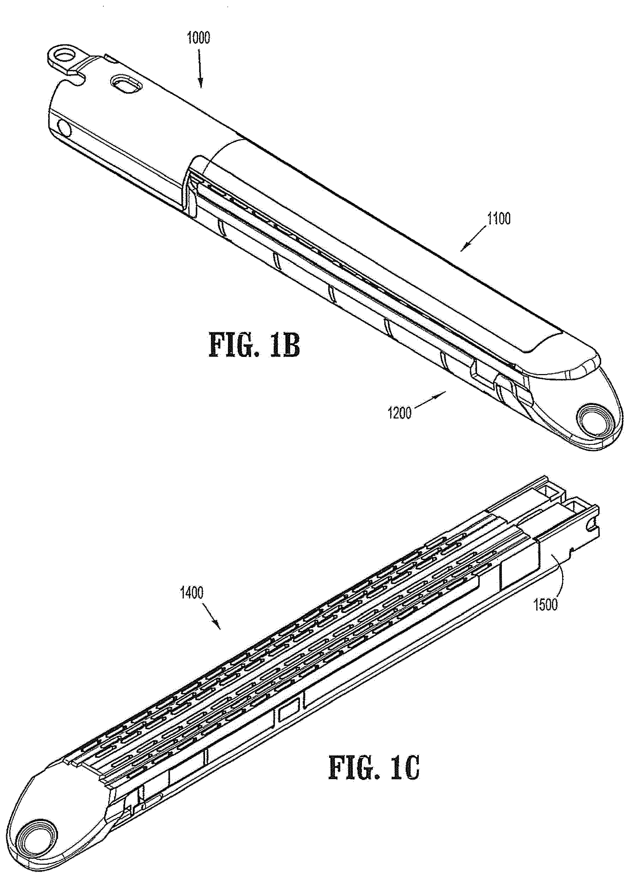

A surgical stapling instrument of the present disclosure is indicated as reference numeral 10 in Figure 1. Additionally, the depicted surgical instrument fires staples, but it may be adapted to fire any other suitable fastener such as clips and two-part fasteners. A loading unit for use with surgical instrument 10 is shown in the accompanying figures and is indicated as reference number 500. A tool assembly of the loading unit 500 is shown in the accompanying figures and is indicated as reference number 1000.

Loading unit 500 is attachable to an elongated or endoscopic portion 18 of surgical instrument 10, e.g., to allow surgical instrument 10 to have greater versatility. Loading unit 500 of the present disclosure is configured for to be used more than once. In particular, the loading unit has a removable assembly 1600 that includes the cartridge assembly 1200. The cartridge assembly 1200 forms a part of the tool assembly 1000, and the tool assembly 1000 forms a portion of the loading unit 500. The removable assembly is configured to be removed and replaced (e.g., after firing fasteners therefrom). Examples of loading units for use with a surgical stapling instrument are disclosed in commonly-owned United States Patent No. 5,752,644 to Bolanos et al. The loading unit 500 shown includes a proximal body portion 502 that is attachable to an endoscopic portion or an elongated portion 18 of a surgical instrument 10 having a handle assembly 12. However, the features of the loading units 500 of the present disclosure, including the tool assembly 1000, can be incorporated in a surgical instrument in which does not include a detachable portion of the elongated portion of the instrument,

Loading unit 500 includes a proximal body portion 502 and a tool assembly 1000. Proximal body portion 502 defines a longitudinal axis "A-A," and is releasably attachable to a distal end of elongated portion 18 of surgical instrument 10. Tool assembly 1000 includes a pair of jaw members including an anvil assembly 1100 and a cartridge assembly 1200. One jaw member is pivotal in relation to the other to enable the clamping of tissue between the jaw members. In the illustrated embodiments, cartridge assembly 1200 is pivotal in relation to anvil assembly 1100 and is movable between an open or undamped position and a closed or approximated position. However, the anvil assembly, or both the cartridge assembly and the anvil assembly, can be movable.

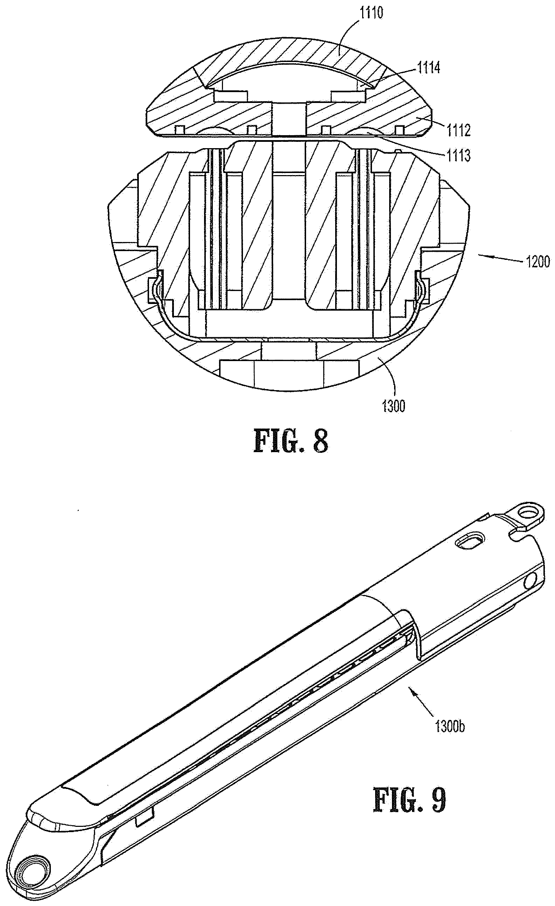

With reference to FIG. 1D, for example, anvil assembly 1100 includes an anvil cover 1110 and an anvil plate 1112, which includes a plurality of staple forming depressions 1113. Anvil plate 1112 is secured to an underside of anvil cover 1110 and defines a channel 1114 (see FIG. 8, for example) therebetween. When tool assembly 1000 is in the approximated position, staple forming depressions 1113 are positioned in juxtaposed alignment with staple receiving slots of the cartridge assembly 1200.

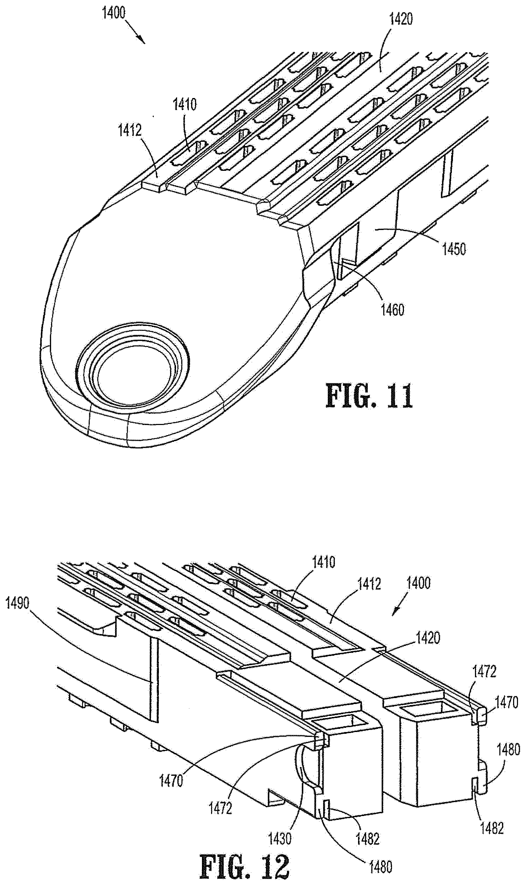

The tool assembly includes a channel or carrier 1300 which receives and supports a cartridge assembly and a support plate 1500. The cartridge assembly has a cartridge body 1400. The cartridge body and support plate 1500 are attached to the channel or carrier 1300 by a snap-fit connection, as discussed below, a detent, latch, or by another type of connection. The cartridge assembly includes fasteners or staples 1414. Cartridge body 1400 defines a plurality of laterally spaced staple retention slots 1410, which are configured as openings in tissue contacting surface 1412 (see FIG. 11). Each slot 1410 is configured to receive a fastener or staple 1414 therein. Cartridge assembly 1200 also defines a plurality of cam wedge slots which accommodate staple pushers 1416 and which are open on the bottom (i.e., away from tissue-contacting surface 1412) to allow an actuation sled 1418 to pass longitudinally therethrough.

Further details of the various components of cartridge assembly 1200, including the connection between its various components, and the removability and replaceability of cartridge body 1400 and support plate 1500 with respect to channel 1300, are discussed below. Generally, the removable assembly 1600 includes cartridge assembly 1200 and support plate 1500. The removable assembly 1600 is removable from channel 1300, e.g., after staples 1414 has been fired from cartridge body 1400. Another removable assembly is capable of being loaded onto channel 1300, such that surgical instrument 10 can be actuated again to fire additional fasteners or staples 1414, for instance.

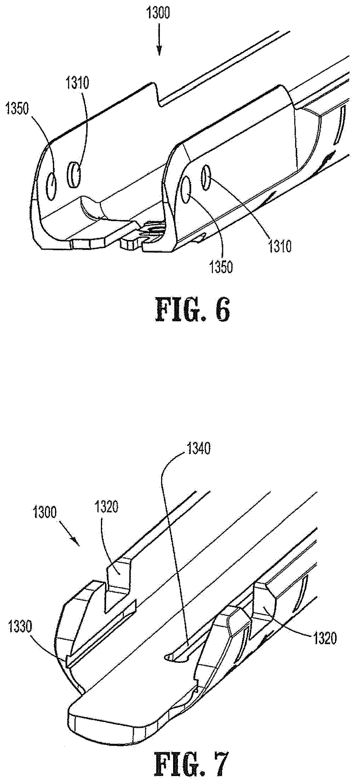

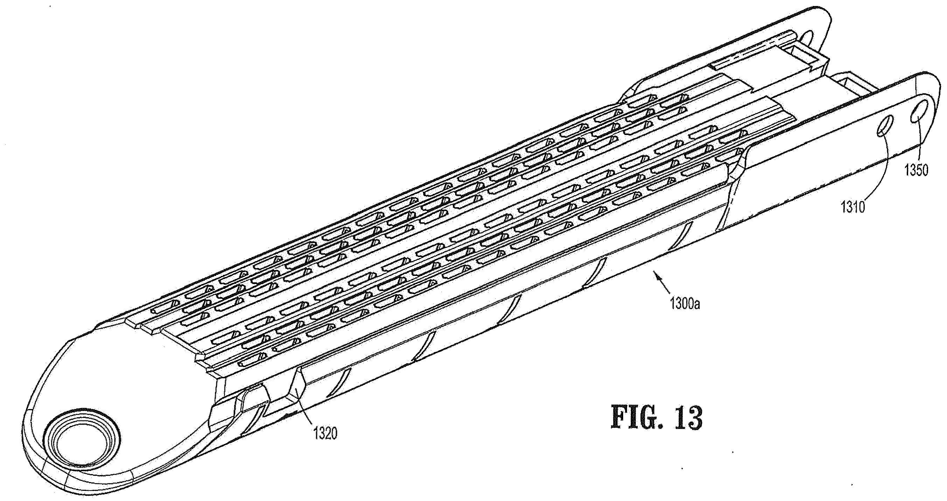

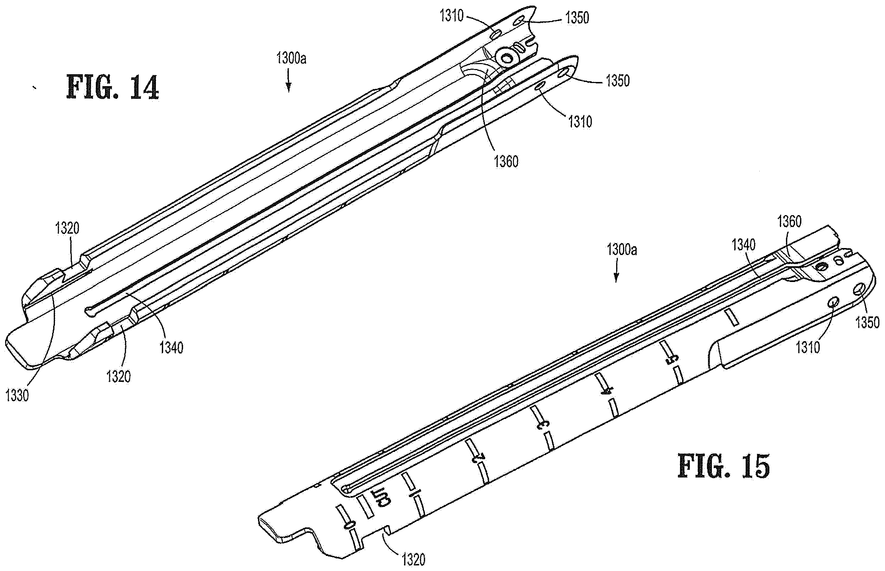

Channel 1300, which may be machined (e.g., e.g., 1300a in FIGS. 13-17) or made of sheet metal (e.g., 1300b in FIG. 9), includes one or a pair of engagement structures or proximal bosses 1310 (e.g., 1300b in FIG. 6), a pair of cut-outs 1320 disposed adjacent a distal end, a pair of distal slots 1330, a central slot 1340, a pair of proximal holes 1350, and a ramped surface 1360. Proximal holes 1350 are configured to align with/mechanically engage a pair of corresponding holes 1120 (e.g., with a pin or protrusion extending through holes 1350 and holes 1120) on anvil cover 1110 to facilitate a pivotal relationship between anvil assembly 1100 and cartridge assembly 1200. It is envisioned that engagement structures 1310 may be pins, protrusions, or similar structure.

Cartridge body 1400 includes a central slot 1420, and rows of staple retention slots 1410 positioned on each side of slot 1420 (see FIG. 11). In the illustrated embodiment, three rows of retention slots 1410 are shown. More specifically, cartridge body 1400 is configured such that actuation sled 1418 can pass through the cam wedge slots and force staple pushers 1416 towards anvil plate 1112. The staples 1414, which are supported on the pushers, are then forced out of their respective staple retention slots 1410. Cartridge body 1400 also includes a pair of engagement structures or U-shaped recesses 1430 (which may, in other embodiments, be slots or openings) adjacent its proximal end, a pair of central bosses 1440, a pair of distal protrusions 1450, and a pair of distal grooves 1460. Pairs of upper and lower mounting surfaces 1470, 1480, respectively, are disposed adjacent a proximal end of cartridge body 1400, and are disposed adjacent respective upper and lower mounting slots 1472, 1482.

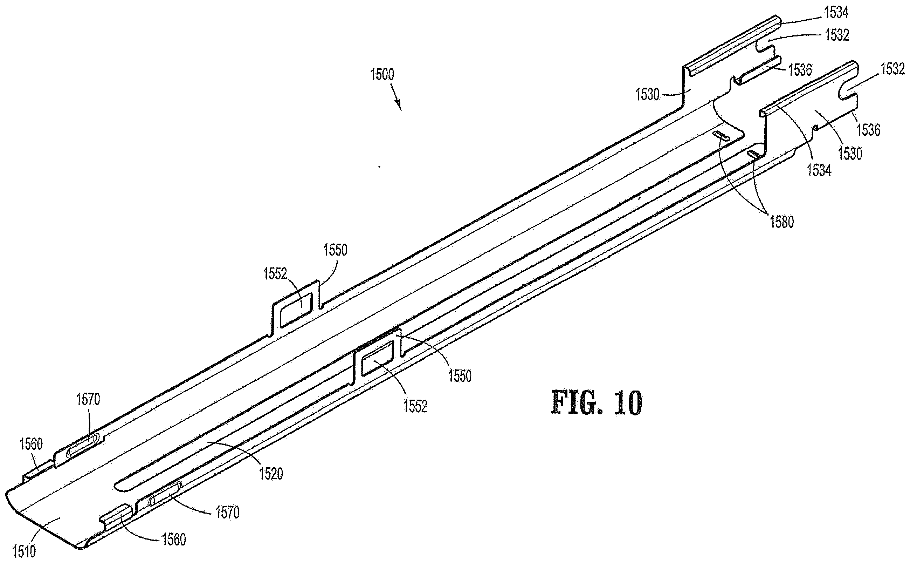

With particular reference to Figure 10, support plate 1500 includes a base surface 1510, a longitudinal slot 1520 extending through base surface 1510, a pair of proximal fingers 1530 disposed and extending substantially perpendicularly from a proximal end of base surface 1510, a pair of intermediate fingers 1550 extending substantially perpendicularly from a middle portion of base surface 1510, a pair of inwardly-extending fingers 1560 and outwardly-extending bosses 1570 disposed adjacent a distal end of base surface 1510, and a pair of proximal protrusions 1580 disposed adjacent the proximal end of base surface 1510. Each proximal finger 1530 includes an engagement structure or proximal-facing U-shaped recesses 1532, an upper mounting flange 1534, and a lower mounting flange 1536. As can be appreciated, support plate 1500 helps maintain pushers 1416 in place with respect to cartridge body 1400. Additionally, longitudinal slot 1520 allows a portion of a drive member to pass through the support plate 1500. The drive member may be a dynamic clamping member 1402. The dynamic clamping member or drive member 1402 drives the actuation sled 1418 through the cartridge body 140. The central slot of the cartridge body, the central slot of the channel, and the longitudinal slot of the support plate are all configured to align with one another to allow the passage of the drive member.

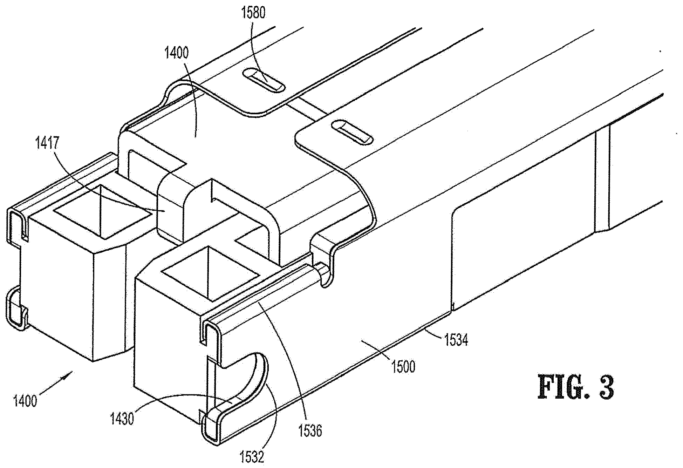

In use, to connect cartridge body 1400 and support plate 1500, cartridge body 1400 and support plate 1500 are assembled or brought together such that the proximal-most end of cartridge is positioned between proximal fingers 1530 of support plate 1500 and in contact with base surface 1510 thereof. Support plate 1500 is then longitudinally translated (e.g., slid distally) with respect to cartridge body 1400 such that upper mounting flanges 1534 and lower mounting flanges 1536 engage upper mounting slots 1472 and lower mounting slots 1482, respectively. The longitudinal translation between cartridge body 1400 and support plate 1500 continues until a distal-most end of proximal fingers 1530 contact a respective vertical wall 1490 (FIG. 12) of cartridge body 1400. At this stage, U-shaped recesses 1430 are laterally adjacent and aligned with U-shaped recesses 1532 (see FIG. 3), and continued proximal movement of cartridge body 1400 with respect to support plate 1500 is prevented. Next, or concomitantly with the relative longitudinal translation between cartridge body 1400 and support plate 1500, cut-outs 1552 within intermediate fingers 1550 of support plate 1500 are positioned around central bosses 1440 of cartridge, and inwardly-extending fingers 1560 are moved into engagement with distal grooves 1460 of cartridge. Cartridge assembly 1200 and support plate 1500 comprise a removable assembly 1600, which is removable from and replaceable onto channel 1300 by the user of the surgical instrument 10 and/or loading unit 500.

Removable assembly 1600 is insertable onto channel 1300 by approximating removable assembly 1600 and channel 1300 such that proximal bosses 1310 are positioned proximally of U-shaped recesses 1430 and 1532, and such that distal ends of distal slots 1330 are positioned proximally of proximal ends of outwardly-extending bosses 1570. Next, removable assembly 1600 is translated longitudinally (e.g., proximally) with respect to channel 1300 such that outwardly-extending bosses 1570 translate proximally within distal slots 1330 until proximal bosses 1310 contact U-shaped recesses 1430 and 1532. Next, or concomitantly with the relative longitudinal translation between removable assembly 1600 and channel 1300, cut-outs 1320 of channel 1300 are moved into engagement with distal protrusions 1450 of cartridge body 1400. Ramped surface 1360 is engaged by the dynamic clamping member 1402 in order to move the anvil assembly 1100 and the cartridge assembly 1200 with respect to one another. A similar surface could be provided on the anvil assembly 1100, in other embodiments. It is envisioned that ramped surface 1360 may also facilitate the alignment and/or engagement between channel 1300 and support plate 1300 and/or cartridge body 1400.

Once assembled, a user is able to actuate movable handle 22 to eject staples 1414 from cartridge body 1400 and into tissue, as described below. It is envisioned that proximal protrusions 1580, which extend from base surface 1510, help maintain actuation sled 1418 in its relative position with respect to support plate 1500 before actuation of instrument 10. That is, it is envisioned that actuation sled 1418, or a portion thereof, is positioned proximally of proximal protrusions 1580, and that proximal protrusions 1580 form a physically barrier to hinder any premature distal advancement of actuation sled 1418. Once a user intends to actuate instrument 10 and distally advance actuation sled 1418 beyond proximal protrusions 1580, the force used to advance actuation sled 1418 is sufficient to force a lower surface or portion of actuation sled 1418 over proximal protrusions 1580.

After staples 1414 have been ejected from cartridge body 1400, and a user wishes to use the same instrument 10 to fire additional staples 1414 (or another type of fastener or knife), the user can remove the removable assembly 1600 by sliding removable assembly 1600 distally with respect to channel 1300. Next, a user removes the removable assembly 1600 from the channel 1300. Another removable assembly with unfired staples can be loaded into the channel 1300. In other embodiments, a cartridge body of a cartridge assembly can be removable from a support plate after the removable assembly is removed from the channel 1300. The cartridge body is removed by sliding support plate 1500 proximally with respect to cartridge body 1400. Another cartridge body, if desired, may be coupled to the support plate and inserted into the channel.

In certain embodiments, the removable assembly is part of a loading unit 500 that is removably attached to the elongated portion of a surgical stapling instrument, such as elongated portion 18. This enables the user to choose a staple line length that is shorter or longer. It is also contemplated that the removable assembly can be used with a surgical instrument that does not have a loading unit that is removable and instead has jaws permanently attached to the elongated portion 18.

During operation of stapler 10, actuation of its movable handle 22 will fire the staples. The handle assembly 12 has an elongate actuation shaft that is translated distally when the movable handle 22 is pivotally moved by the user. The actuation shaft of the handle assembly can include teeth that are engaged by the movable handle 22, or the handle assembly 12 can include a series of gears for moving the actuation shaft. Alternatively, the handle assembly can include a motorized driver for moving the actuation shaft, or the handle assembly can be attachable to a separate motorized driver.

In certain embodiments, through successive strokes of the movable handle, a drive rod 30 (a distal portion of which is illustrated in Figures 1 and 27-31)) is advanced distally, such that drive rod 30 pushes a portion of the drive assembly (which includes the dynamic clamping member 1402) to translate distally through cartridge body 1400. (Further details of how actuation of movable handle 22 causes distal advancement of drive rod 30 are explained in U.S. Patent No. 6,953,139 to Milliman et al.) Distal movement of the drive assembly, and in particular, the dynamic clamping member or drive member 1402, causes approximation of one jaw member with respect to the other. That is, an upper portion of the dynamic clamping member 1402 travels through the channel 1114 between the anvil plate 1112 and the anvil cover 1110, and a lower portion of the dynamic clamping member 1402 travels below the carrier 1300 of the cartridge assembly 1200, which causes approximation of the anvil assembly 1100 and the cartridge assembly 1200 to clamp tissue therebetween. For example, the channel 1300 may have a lower surface defining a camming surface and the lower portion of the dynamic clamping member 1402 engages the camming surface to pivot the cartridge assembly 1200 toward the anvil assembly 1100.

Additionally, distal translation of the dynamic clamping member 1402 causes the actuation sled 1418 to move distally through cartridge body 1400, which causes cam wedges 1419 of actuation sled 1418 to sequentially engage pushers 1416 to move pushers 1416 vertically within staple retention slots 1410 and eject staples 1414 into staple forming depressions 1113 of anvil plate 1112. Subsequent to the ejection of staples 1414 from retention slots 1410 (and into tissue), a cutting edge of the dynamic clamping member 1402 severs the stapled tissue as the cutting edge travels distally through central slot 1420 of cartridge body 1400.

It is also envisioned, in further embodiments, that an end effector or tool assembly like the end effector or tool assembly 1000 is arranged for articulating between a first position where tool assembly 1000 is aligned with longitudinal axis "A-A," and a second position where tool assembly 1000 is disposed at an angle with respect to longitudinal axis "A-A." For example, the anvil assembly 110 may be pivotably attached to the proximal body portion 502 of a loading unit 500, or pivotably attached to the elongated portion of the instrument. The loading unit includes one or more cables or linkages disposed in the proximal body portion 502 and attached at the tool assembly 1000. When the cable or linkage is displaced, the tool assembly pivots and articulates with respect to the instrument. Further details of providing articulation are described in detail in commonly-owned U.S. Patent No. 6,953,139 to Milliman et al. Further, the tool assembly can be configured not to articulate.

Additionally, it is envisioned that instrument 10 is powered by a power source and/or motor. Further details of such a powered surgical instrument are included in U.S. Patent Publication No. 2008/0255607.

Further, and as illustrated in Figure 11, for example, the present disclosure includes a cartridge body 1400 having a stepped tissue-contacting surface 1412. In such an embodiment, different sized staples 1414, or all the same sized staples, may be used. Further details of a staple cartridge having multiple staple sizes are included in U.S. Patent No. 7,407,075 to Holsten et al..

The present disclosure also relates to methods of using the described surgical instrument 10, loading unit 500, and tool assembly 100 to perform a surgical procedure and to methods of assembling the various components thereof, as described above.

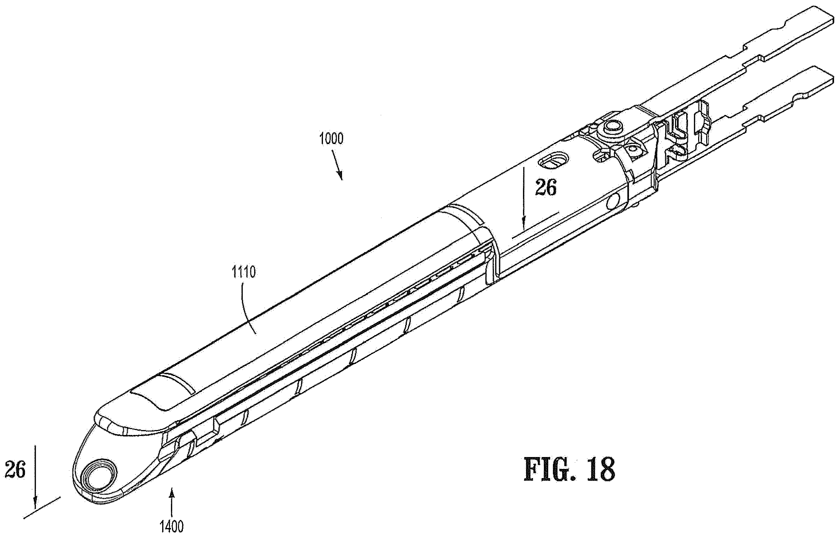

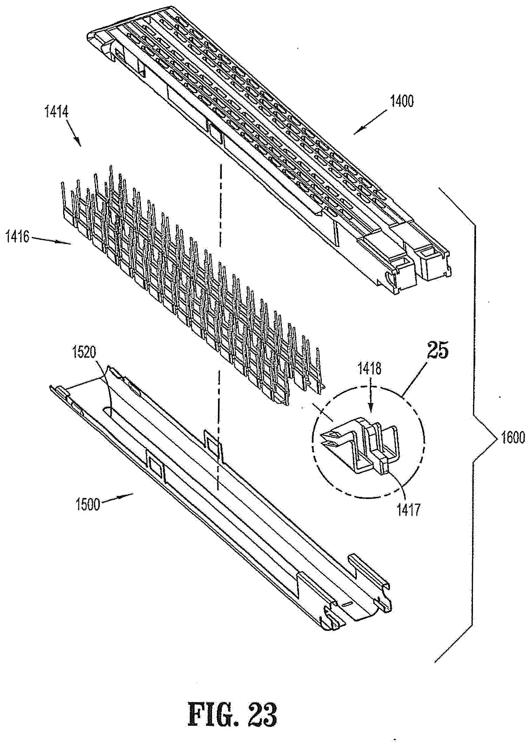

With reference to Figures 18-35, two embodiments of a lockout mechanism 2000, 2000a of the present disclosure are shown. For each of these embodiments, a surgical instrument having the lockout may have a channel, removable assembly, cartridge body, support plate, and the engagement structures discussed above. Furthermore, the present disclosure is directed to a removable assembly having the lockout, or a loading unit having the lockout.

With reference to Figures 18-31, the first embodiment of lockout mechanism 2000 includes a latch 2010 and a spring 2030, and is configured to prevent refiring of cartridge body 1400 of removable assembly 1600, and also prevent distal translation of dynamic clamping member 1402 after an initial distal translation of knife and prior to another removable assembly 1600 being loaded onto channel 1300.

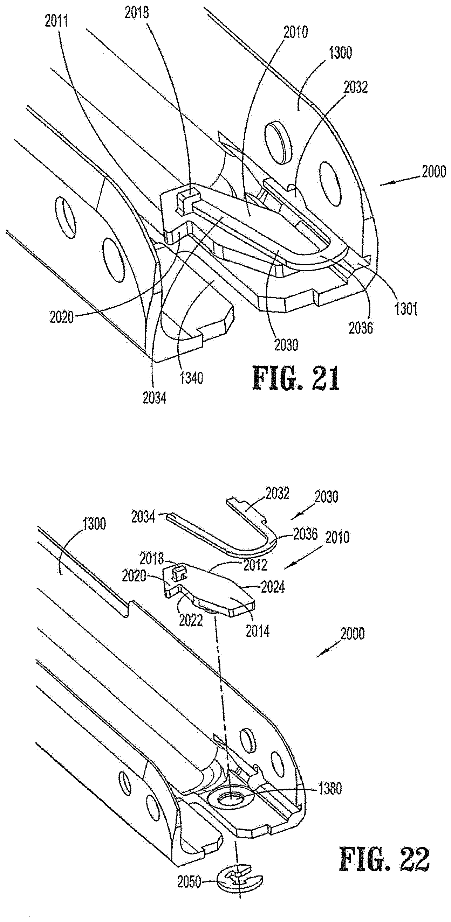

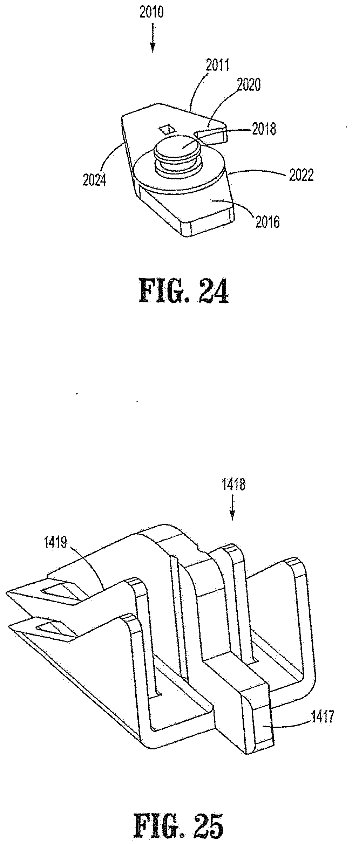

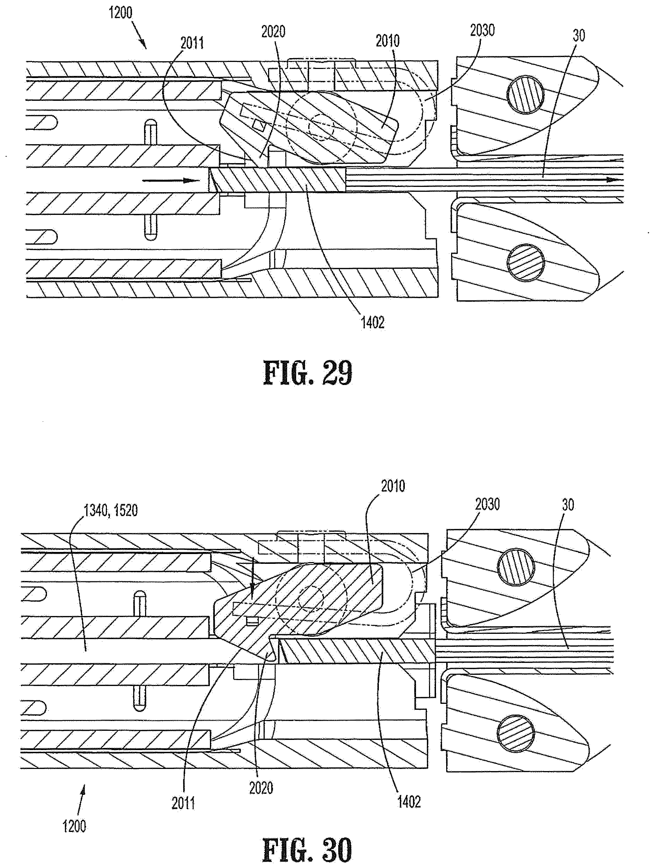

With particular reference to Figures 22 and 24, latch 2010 includes a body 2012 having an upper surface 2014 and a lower surface 2016, a lower protrusion 2018 depending downwardly from lower surface 2016, a spring stop 2019 extending upwardly from upper surface 2014, and a shaped surface 2020 on a first lateral side 2022. The body 2012 also has a second lateral side 2024. The shaped surface 2020 has two sides. The first side 2020a is angled with respect to the central slot 1340 when the latch 2010 is in a blocking position in which the latch obstructs the passage of the dynamic clamping member 1402. The second side 2020b of the shaped surface 2020 extends transversely to the central slot 1340 when the latch is in the blocking position. (See Fig. 30).

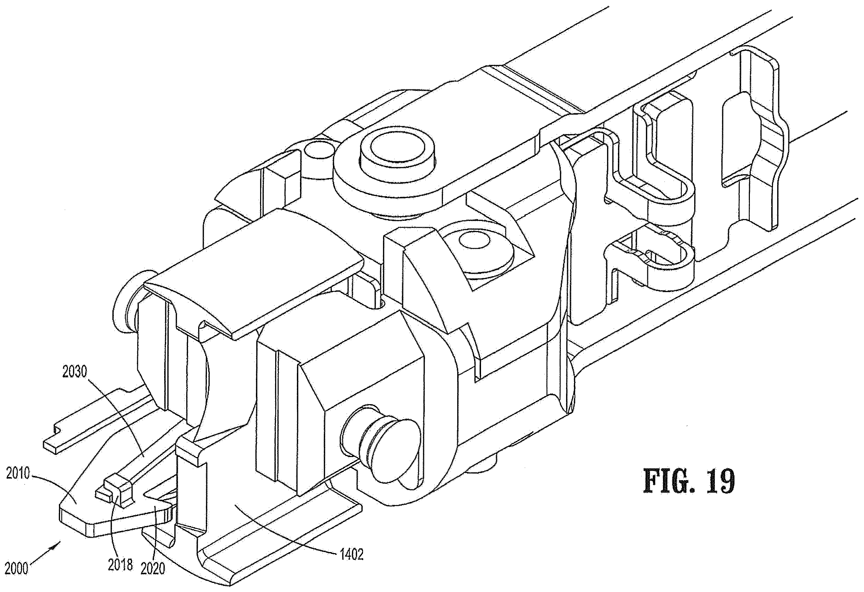

Referring now to Figures 19-24, latch 2010 is mechanically engaged with channel 1300 so that the latch 2010 can pivot with respect to the channel 1300. In particular, lower protrusion 2018 of latch 2010 (Figure 24) extends through an opening 1380 (Figure 22) in channel 1300, such that latch 2010 is pivotable with respect to channel 1300. Lower protrusion 2018 is maintained in mechanical engagement with channel 1300 by a lock pin 2050 (Figure 22). Alternatively, the protrusion can be omitted and a separate pivot pin in engagement with the body 2012 and the channel 1300 can be used.

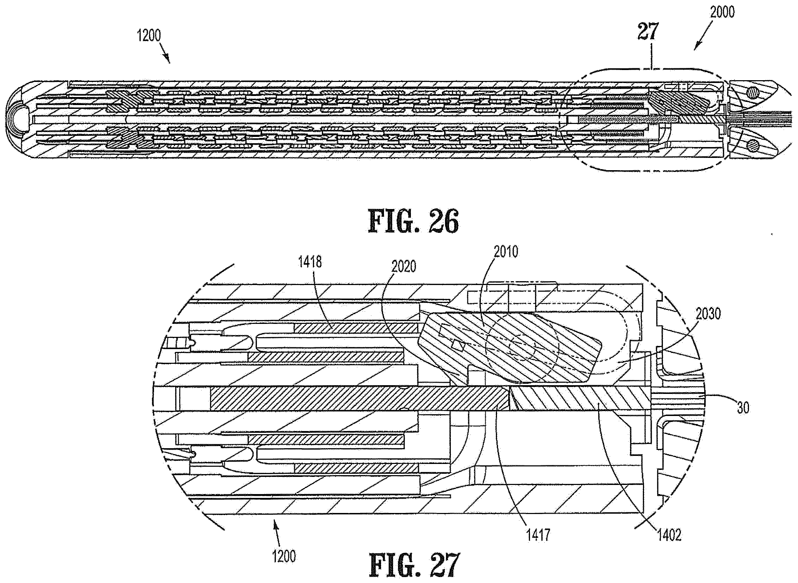

With regard to Figures 21-22, spring 2030 includes a first leg 2032, a second leg 2034, and an intermediate portion 2036 interconnecting first leg 2032 and second leg 2034. First leg 2032 is in contact with a portion of channel 1300. For example, the channel 1300 may have a slot 1301, a notch, or some other feature for restricting the movement of the first leg 2032. Second leg 2034 is disposed in contact with spring stop 2018 of latch 2010. Intermediate portion 2036 is disposed between first leg 2032 and second leg 2034. For example, the spring may have a U-shaped configuration (see Fig. 27), or some other shape, such as L-shaped.

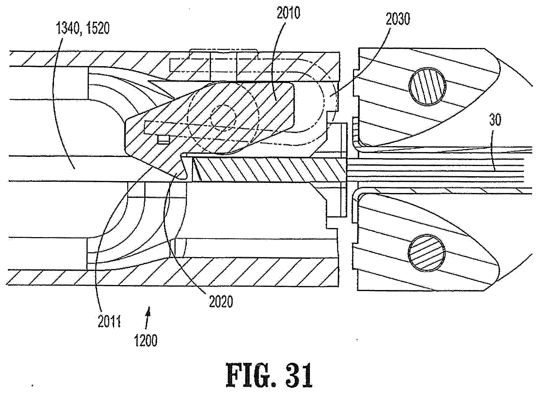

Spring 2030 is in mechanical cooperation with a portion of the cartridge assembly 1200. The spring is configured to bias latch 2010 towards its blocking position. In the initial position of the dynamic clamping member 1402 and the sled 1418 (e.g., prior to distal advancement thereof to fire staples and incise tissue), a tail portion 1417 of sled 1418 (Figure 25) physically prevents the shaped surface 2020 of latch 2010 from moving from its initial position into its blocking position, and thus allows distal translation of dynamic clamping member 1402 and sled 1418 (see Figures 26 and 27). After translation of the dynamic clamping member and sled, the spring moves the latch 2010 to the blocking position, where the shaped surface 2020 of latch 2010 obstructs the central slot 1340 of channel 1300 and the longitudinal slot 1520 extending through base surface 1510 of support plate 1500 (see Figures 30 and 31), such that shaped surface 2020 would block distal translation of dynamic clamping member 1402 when the dynamic clamping member 1402 has been retracted after firing staples and cutting tissue.

The latch 2010 is laterally movable from an initial position to a blocking position. The latch moves laterally, which enables the shaped surface of the latch to obstruct the slot and move away from a position that obstructs the slot of the cartridge assembly.

During retraction of the dynamic clamping member, the dynamic clamping member slides along the shaped surface first side 2020a, keeping the latch 2010 away from the dynamic clamping member and pivoting the latch against the bias of the spring. In the retracted position of the dynamic clamping member, it is disposed proximally of shaped surface 2020 and the sled 1418 and/or tail portion 1417 is not abutting the shaped surface 2020. The latch 2010 pivots to the blocking position, so that the second side 2020b obstructs and/or prevents distal movement of the dynamic clamping member.

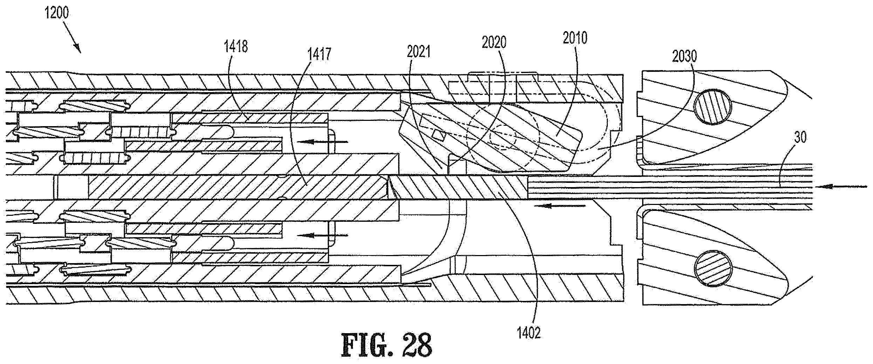

During distal advancement of dynamic clamping member 1402 and sled 1418, and after sled 1418 distally passes latch 2010 such that shaped surface 2020 is no longer in contact with tail portion of sled 1418, dynamic clamping member 1402 abuts the shaped surface 2020, which physically blocks latch 2010 from moving into its blocking position, and thus permits distal translation of dynamic clamping member 1402 (see Figure 28).

When cartridge assembly 1200 or removable assembly 1600 is removed from channel 1300, latch 2010 continues to block dynamic clamping member 1402 (see Figure 31). When a new cartridge assembly 1200 or removable assembly 1600 is loaded onto channel 1300, tail portion 1417 of the new sled 1418 engages shaped surface first side 2020a of latch 2010 and pivots latch 2010 away from its blocking position. Without a sled having the correct configuration, the latch remains in the blocking position.

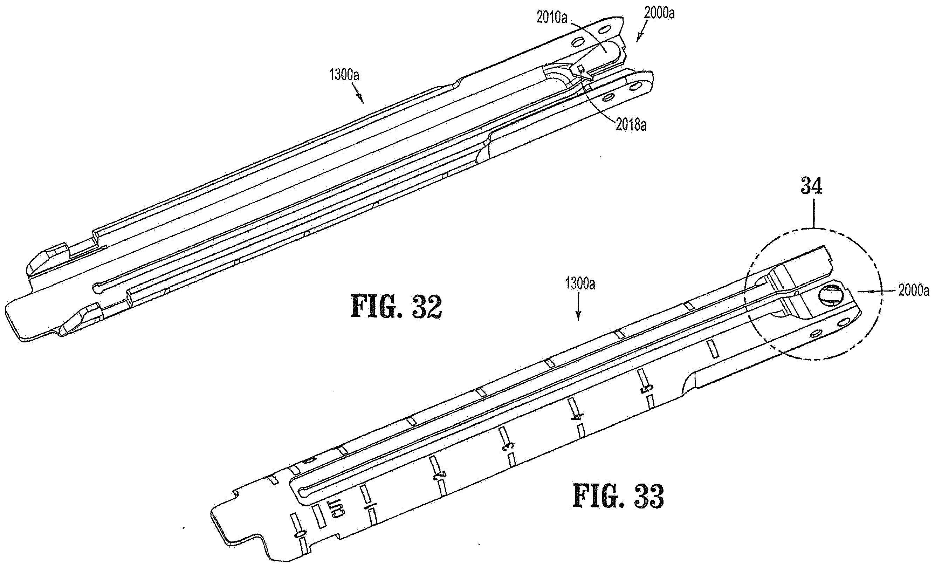

With reference to Figures 32-35, a second embodiment of lockout mechanism 2000a is shown. A surgical instrument having the lockout may have a channel, removable assembly, cartridge body, support plate, and the engagement structures discussed above. Furthermore, the present disclosure is directed to a removable assembly having the lockout, or a loading unit having the lockout. Lockout mechanism 2000a includes a latch 2010a, and a spring. The spring is not shown for clarity, but may be as discussed above. Unlike the embodiment of lockout mechanism 2000 discussed above, this embodiment of lockout mechanism 2000a does not include a lock pin 2050. Here, to maintain latch 2010a in engagement with channel 1300a, lower surface 2016a of latch 2010a includes a locking member 2018a depending therefrom.

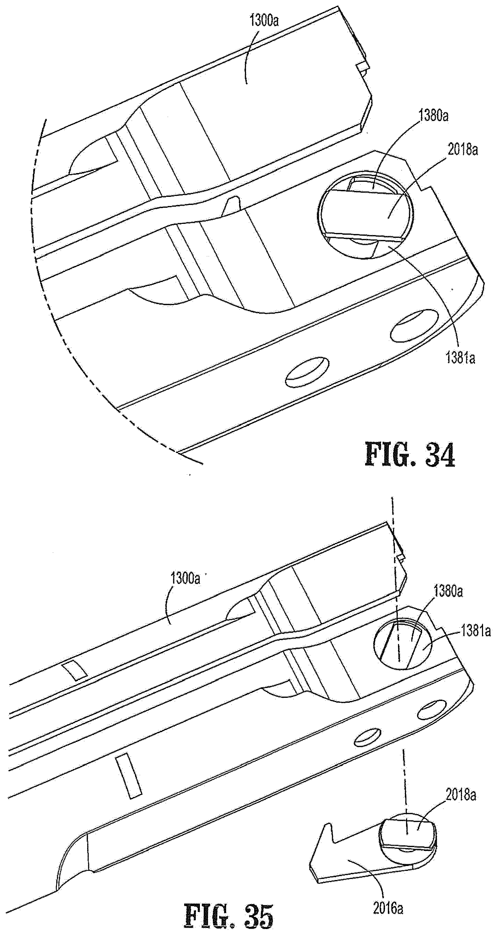

In the illustrated embodiment, locking member 2018a includes a pair of parallel walls that are interconnected by a pair of arcuate walls. The opening 1380a of channel 1300a includes similar, but slightly larger shape with respect to locking member 2018a and also includes a circular recess 1381a, around which locking member 2018a can rotate (see Figures 34 and 35).

To engage latch 2010a with channel 1300a, locking member 2018a is inserted through opening 1380a and latch 2010a is then rotated a predetermined amount (e.g., about 40 degrees to about 130 degrees) such that latch 2010a does not fall through opening 1380a of channel 1300a. The spring (not shown in this embodiment for clarity) may then be positioned between spring stop 2018a of latch 2010a and a portion of channel 1300a, as described above.

As can be appreciated, use of surgical instrument including the second embodiment of lockout mechanism 2000a is similar to, or that same as use of the surgical instrument including the first embodiment of lockout mechanism 2000, as described above.

While the above description contains many specifics, these specifics should not be construed as limitations on the scope of the present disclosure, but merely as illustrations of various embodiments thereof. Therefore, the above description should not be construed as limiting, but merely as exemplifications of various embodiments.

## Claims

1. A surgical instrument (10), comprising:
a channel (1300) including an engagement structure (1310) disposed adjacent a proximal end thereof; and
a removable assembly (1600) disposed in releasable engagement with the channel, the removable assembly comprising:
a cartridge body (1400) configured to house a plurality of fasteners (1414) therein, the cartridge body including an engagement structure (1430) disposed adjacent a proximal end thereof; and
a support plate (1500) configured to mechanically engage the cartridge body, the support plate including an engagement structure (1532) disposed adjacent a proximal end thereof;
wherein the engagement structure of the cartridge body is configured for longitudinal alignment with the engagement structure of the support plate, and wherein the engagement structure of the cartridge body and the engagement structure of the support plate are configured to mechanically engage the engagement structure of the channel when the removable assembly is engaged with the channel, **characterised in that** the engagement structure of the channel includes at least one raised boss.

2. The surgical instrument of Claim 1, wherein the engagement structure of the cartridge body includes at least one U-shaped recess.

3. The surgical instrument of Claim 2, wherein the engagement structure of the support plate includes a U-shaped recess.

4. The surgical instrument of Claim 3, wherein each of the at least one U-shaped recess of the cartridge body and the at least one U-shaped recess of the support plate include a proximally-facing opening.

5. The surgical instrument of any preceding claim, wherein the channel includes a longitudinally-extending slot disposed adjacent a distal end thereof, and wherein the support plate includes an outwardly-extending finger (1530) configured to releasably engage the longitudinally-extending slot of the channel.

6. The surgical instrument of any preceding claim, wherein the support plate includes an inwardly-extending finger (1560) disposed on a distal portion thereof, the inwardly-extending finger configured to releasably engage a groove (1460) disposed on a distal portion of the cartridge body.

7. The surgical instrument of any preceding claim, wherein the support plate includes a proximal protrusion (1580) disposed adjacent a proximal end thereof, the proximal protrusion being configured to help prevent an actuation sled (1418) from prematurely translating distally with respect to the cartridge body.

8. The surgical instrument of any preceding claim, wherein the channel is part of a removable loading unit that includes an anvil assembly.

9. The surgical instrument of Claim 8 further comprising a body portion to which the channel and the anvil assembly are attached, the body portion being attachable to the elongate member of a surgical instrument.

## Patentansprüche

1. Chirurgisches Instrument (10), umfassend:
einen Kanal (1300) mit einer Eingriffsstruktur (1310), die neben einem proximalen Ende davon angeordnet ist; und
eine abnehmbare Anordnung (1600), die in lösbarem Eingriff mit dem Kanal angeordnet ist, wobei die abnehmbare Anordnung umfasst:
einen Kartuschenkörper (1400), der zur Aufnahme einer Vielzahl von Befestigungselementen (1414) konfiguriert ist, wobei der Kartuschenkörper eine Eingriffsstruktur (1430) aufweist, die neben einem proximalen Ende davon angeordnet ist; und
eine Trägerplatte (1500), die zum mechanischen Eingriff mit dem Kartuschenkörper konfiguriert ist, wobei die Stützplatte eine Eingriffsstruktur (1532) aufweist, die neben einem proximalen Ende davon angeordnet ist;
wobei die Eingriffsstruktur des Kartuschenkörpers zur Längsausrichtung mit der Eingriffsstruktur der Trägerplatte konfiguriert ist, und wobei die Eingriffsstruktur des Kartuschenkörpers und die Eingriffsstruktur der Trägerplatte so konfiguriert sind, dass sie mechanisch mit der Eingriffsstruktur des Kanals in Eingriff kommen, wenn die entfernbare Anordnung mit dem Kanal in Eingriff ist, **dadurch gekennzeichnet dass** die Eingriffsstruktur des Kanals mindestens einen erhöhten Vorsprung aufweist.

2. Chirurgisches Instrument nach Anspruch 1, wobei die Eingriffsstruktur des Kartuschenkörpers mindestens eine U-förmige Aussparung aufweist.

3. Chirurgisches Instrument nach Anspruch 2, wobei die Eingriffsstruktur der Trägerplatte eine U-förmige Aussparung aufweist.

4. Chirurgisches Instrument nach Anspruch 3, wobei die mindestens eine U-förmige Aussparung des Kartuschenkörpers und die mindestens eine U-förmige Aussparung der Trägerplatte jeweils eine proximal gerichtete Öffnung aufweisen.

5. Chirurgisches Instrument eines der vorstehenden Ansprüche, wobei der Kanal einen sich in Längsrichtung erstreckenden Schlitz aufweist, der neben einem distalen Ende desselben angeordnet ist, und wobei die Trägerplatte einen sich nach außen erstreckenden Finger (1530) aufweist, der so konfiguriert ist, dass er lösbar in den sich in Längsrichtung erstreckenden Schlitz des Kanals eingreift.

6. Chirurgisches Instrument eines der vorstehenden Ansprüche, wobei die Trägerplatte einen sich nach innen erstreckenden Finger (1560) aufweist, der an einem distalen Abschnitt desselben angeordnet ist, wobei der sich nach innen erstreckende Finger so konfiguriert ist, dass er lösbar in eine Nut (1460) eingreift, die an einem distalen Abschnitt des Kartuschenkörpers angeordnet ist.

7. Chirurgisches Instrument eines der vorstehenden Ansprüche, wobei die Trägerplatte einen proximalen Vorsprung (1580) aufweist, der neben einem proximalen Ende desselben angeordnet ist, wobei der proximale Vorsprung so konfiguriert ist, dass er verhindert, dass sich ein Betätigungsschlitten (1418) vorzeitig distal in Bezug auf den Kartuschenkörper verschiebt.

8. Chirurgisches Instrument eines der vorstehenden Ansprüche, wobei der Kanal Teil einer abnehmbaren Ladeeinheit ist, die eine Ambossanordnung aufweist.

9. Chirurgisches Instrument nach Anspruch 8, das weiter einen Körperteil umfasst, an dem der Kanal und die Ambossanordnung befestigt sind, wobei der Körperteil an dem länglichen Teil eines chirurgischen Instruments befestigt werden kann.

## Revendications

1. Instrument chirurgical (10), comprenant :
un canal (1300) incluant une structure de mise en prise (1310) disposée adjacente à une extrémité proximale de celui-ci ; et
un ensemble amovible (1600) disposé dans une mise en prise pouvant être libérée avec le canal, l'ensemble amovible comprenant :
un corps de cartouche (1400) configuré pour loger une pluralité d'attaches (1414) en son sein, le corps de cartouche incluant une structure de mise en prise (1430) disposée adjacente à une extrémité proximale de celui-ci ; et
une plaque de support (1500) configurée pour mettre en prise mécaniquement le corps de cartouche, la plaque de support incluant une structure de mise en prise (1532) disposée adjacente à une extrémité proximale de celle-ci ;
dans lequel la structure de mise en prise du corps de cartouche est configurée pour un alignement longitudinal avec la structure de mise en prise de la plaque de support, et dans lequel la structure de mise en prise du corps de cartouche et la structure de mise en prise de la plaque de support sont configurées pour mettre en prise mécaniquement la structure de mise en prise du canal lorsque l'ensemble amovible est mis en prise avec le canal, **caractérisé en ce que** la structure de mise en prise du canal inclut au moins une protubérance élevée.

2. Instrument chirurgical selon la revendication 1, dans lequel la structure de mise en prise du corps de cartouche inclut au moins une partie en retrait en forme de U.

3. Instrument chirurgical selon la revendication 2, dans lequel la structure de mise en prise de la plaque de support inclut une partie en retrait en forme de U.

4. Instrument chirurgical selon la revendication 3, dans lequel chacune de l'au moins une partie en retrait en forme de U du corps de cartouche et de l'au moins une partie en retrait en forme de U de la plaque de support inclut une ouverture faisant face de façon proximale.

5. Instrument chirurgical selon l'une quelconque des revendications précédentes, dans lequel le canal inclut une fente s'étendant de façon longitudinale disposée adjacente à une extrémité distale de celui-ci, et dans lequel la plaque de support inclut un doigt s'étendant vers l'extérieur (1530) configuré pour mettre en prise de façon à pouvoir être libérée la fente s'étendant de façon longitudinale du canal.

6. Instrument chirurgical selon l'une quelconque des revendications précédentes, dans lequel la plaque de support inclut un doigt s'étendant vers l'intérieur (1560) disposé sur une portion distale de celle-ci, le doigt s'étendant vers l'intérieur étant configuré pour mettre en prise de façon à pouvoir être libérée une rainure (1460) disposée sur une portion distale du corps de cartouche.

7. Instrument chirurgical selon l'une quelconque des revendications précédentes, dans lequel la plaque de support inclut une protubérance proximale (1580) disposée adjacente à une extrémité proximale de celle-ci, la protubérance proximale étant configurée pour aider à empêcher un chariot d'actionnement (1418) de prématurément subir une translation de façon distale par rapport au corps de cartouche.

8. Instrument chirurgical selon l'une quelconque des revendications précédentes, dans lequel le canal fait partie d'une unité de chargement amovible qui inclut un ensemble enclume.

9. Instrument chirurgical selon la revendication 8 comprenant en outre une portion formant corps à laquelle le canal et l'ensemble enclume sont attachés, la portion formant corps pouvant être attachée à l'élément allongé d'un instrument chirurgical.
